(19) Europäisches Patentamt

European Patent Office

Office européen des brevets

(11) EP 0 684 941 B1

## (12) EUROPÄISCHE PATENTSCHRIFT

(45) Veröffentlichungstag und Bekanntmachung des Hinweises auf die Patenterteilung:
**29.10.1997 Patentblatt 1997/44**

(21) Anmeldenummer: 94906087.5

(22) Anmeldetag: 17.02.1994

(51) Int. Cl.$^6$: **C07C 51/47**

(86) Internationale Anmeldenummer:
**PCT/AT94/00016**

(87) Internationale Veröffentlichungsnummer:
**WO 94/19307 (01.09.1994 Gazette 1994/20)**

(54) **VERFAHREN ZUR ABTRENNUNG UND REINIGUNG VON MILCHSÄURE**

LACTIC ACID EXTRACTION AND PURIFICATION PROCESS

PROCEDE D'EXTRACTION ET DE PURIFICATION D'ACIDE LACTIQUE

(84) Benannte Vertragsstaaten:
**DE FR GB IT NL**

(30) Priorität: **18.02.1993 AT 310/93**

(43) Veröffentlichungstag der Anmeldung:
**06.12.1995 Patentblatt 1995/49**

(73) Patentinhaber: **VOGELBUSCH GESELLSCHAFT m.b.H.**
**A-1050 Wien (AT)**

(72) Erfinder:
• **SARHADDAR, Schahroch**
**A-1060 Wien (AT)**
• **SCHEIBL, Anton**
**A-1150 Wien (AT)**
• **BERGHOFER, Emmerich**
**A-3021 Pressbaum (AT)**
• **CRAMER, Adalbert**
**A-4661 Roitham (AT)**

(74) Vertreter: **Itze, Peter, Dipl.-Ing. et al**
**Patentanwälte**
**Casati, Wilhelm, Dipl.-Ing.**
**Itze, Peter, Dipl.-Ing.**
**Amerlingstrasse 8**
**1061 Wien (AT)**

(56) Entgegenhaltungen:
BE-A- 878 531          FR-A- 2 455 022

• CHEMICAL ABSTRACTS, vol. 78, no. 21, 28. Mai 1973, Columbus, Ohio, US; abstract no. 135586b, NAPIERALA, W. ET AL. 'Production of alimentary lactic acid of high purity' Seite 326 ; & PRZEM. FERMENT. ROLNY Bd. 16, Nr. 12 , 1972 Seiten 4 - 10
• PATENT ABSTRACTS OF JAPAN vol. 12, no. 110 (C-486)8. April 1988 & JP,A,62 238 232 (RESEARCH ASSOCIATION FOR THE UTILIZATION OF LIGHT OIL) 19. Oktober 1987
• CHEMICAL ABSTRACTS, vol. 111, no. 9, 28. August 1989, Columbus, Ohio, US; abstract no. 76533p, 'Purification of lactic acid from fermentation broth' Seite 628 ; & JP,A,1 091 788 (SHIMADZU CORPORATION) 11. April 1989

**Beschreibung**

Die Erfindung bezieht sich auf ein Verfahren zur Abtrennung und Reinigung von Milchsäure aus salz- und kohlenhydrathaltigen, von grobdispersen und lipophilen Verunreinigungen befreiten Substraten (Fermentationslösung).

Die großtechnische Herstellung von Milchsäure, vor allem wenn reine L(+)- bzw. D(-)-Milchsäure gewonnen werden soll, erfolgt heute im überwiegendem Maße durch biotechnologische Verfahren. Dabei unterteilt sich der Herstellungsprozeß:

1) in die eigentliche Herstellung der Milchsäure durch Fermentation eines kohlenhydrathältigen

2) in die Aufarbeitung (down stream processing) der Fermentationslösung zur reinen Säure.

Die Industrie hat sich eine ganze Reihe von Mikroorganismen-Stämme zur Produktion von Milchsäure zu eigen gemacht. Von Bedeutung sind dabei vor allem die homofermentativen Milchsäurebakterien der Gattungen Lactobacillus, Streptococcus und Pediococcus, die aber nur in einem sehr engem pH-Bereich ihre maximale Produktivität erreichen.

Es ist daher notwendig, während der Fermentation nicht nur die für den gewählten Organismus optimale Temperatur, sondern auch den erforderlichen pH-Wert konstant zu halten. Aus diesem Grund werden der Maische vor und/oder während der Fermentation Neutralisationsmittel, wie z.B. Alkalihydroxide, Ca-Carbonat, Kalkmilch oder Ammoniumwasser, zuzugeben, sodaß eine Übersäuerung vermieden, und ständig ein pH-Wert von 5,5 - 6,5 aufrechterhalten wird.

Die Fermentationsmaische enthält also als Hauptbestandteil das Salz der Milchsäure (z.B. $NH_4$-Lactat oder Ca-Lactat) neben wenig freier Säure, sowie nicht umgesetzte Ausgangsstoffe (z.B. Zucker), Schwermetalle, Farbstoffe, Stoffwechselnebenprodukte (z.B. Essigsäure), Zellen und Zellfragmente der Mikroorganismen sowie anorganische Salze.

Eine direkte Verwendung der aus der Fermentation kommenden Lösung ist daher nicht möglich, und zur Gewinnung der reinen freien Milchsäure sind noch weitere Aufarbeitungsschritte erforderlich.

Zur Abtrennung und Gewinnung von freier Milchsäure aus der Fermentationsmaische werden mehrere Methoden beschrieben.

Als einfachstes und von den meisten Produzenten angewandtes großtechnisches Reinigungsverfahren hat sich die Fällung der Milchsäure als Ca-Lactat erwiesen. Bei diesem Verfahren wird nach Beendigung des Fermentationsprozesses zunächst die Maische auf ca. 80 - 90° C erhitzt, und der pH-Wert auf 10 - 11 erhöht. Durch diesen Schritt werden die Mikroorganisen abgetötet, die Proteine koaguliert, und das gebildete Ca-Lactat gelöst. Nach Abtrennung aller unlöslichen Bestandteile wird die Maische zur Freisetzung der Milchsäure aus ihrem Salz mit Schwefelsäure angesäuert. Um auch die insbesonders durch Korrosion eingeschleppten Fe- und Cu-Ionen zu entfernen, wird noch Na- oder Ca-hexacyanoferrat (II) zugegeben, und die ausfallenden Ferrocyanidsalze zusammen mit dem Gips über ein Drehfilter oder eine Filterpresse abgetrennt. Färbende Bestandteile werden durch Aktivkohle entfernt. Die erhaltene Dünnsäure wird anschließend auf eine ca. 80 %ige Milchsäure aufkonzentriert, wobei gleichzeitig geringe Mengen mitentstandener, flüchtiger Säuren abgetrennt werden.

Bei verbesserten Fällungs-Verfahren wird die Rohmilchsäure zunächst mit Aktivkohle entfärbt und zur restlosen Entfernung noch enthaltener Salze können Reinigungsschritte über Kationenaustauscher nachgeschaltet werden. Die erhaltene kationenfreie Lösung kann nun entweder direkt der Eindampfung bzw. Kristallisation zugeführt oder weiter über einen Anionenaustauscher geleitet werden, um noch vorhandene Fremdanionen, hauptsächlich Sulfat- und Chloridionen, zu entfernen (DD-PS 6740).

Zur weiteren Qualitätsverbesserung, insbesonders des Geruchs und Geschmacks, wird manchmal auch eine oxidative Behandlung mit Wasserstoffperoxid oder Kaliumpermanganat angeschlossen. (CARLOS BELLAPART VILA, 1964, ES 297969)

Alle Fällungsverfahren haben jedoch schwerwiegende Nachteile: Diese sind, neben dem relativ hohen technischen Aufwand, vor allem die durch den Fällungs- bzw. Kristallisationsprozeß verursachten Substanzverluste, die bis zu 20 % der Milchsäure betragen können (HEDING, L.G., Biotechm. Bioeng. 17, 1975, 1363 - 1364). Darüber hinaus werden bei diesen Verfahren große Mengen an Hilfschemikalien benötigt. Da in den meisten Fällen die Milchsäure als Ca-Lactat anfällt, welches erst mit Schwefelsäure zur freien Säure und einer äquivalenten Menge Gips umgesetzt werden muß, fallen nicht nur die Kosten für Kalk und Schwefelsäure an, sondern auch jene zur Entsorgung großer Gipsmengen.

Die nach diesem "Fällungsverfahren" herstellbare Milchsäurequalität ist "Genußmilchsäure" und damit nur für die Lebensmittelindustrie geeignet. Für die Pharmazie wird allerdings Milchsäure höherer Reinheit benötigt. Soll aus Milchsäure durch Polymerisation abbaubare Kunststoffe erzeugt werden, sind noch höhere Reinheitskriterien erforderlich. Insbesondere ist dabei eine vollkommene Abwesenheit von Kohlenhydraten notwendig. Es hat daher nicht an Versuchen gefehlt, die Milchsäure auf anderem Wege aus der Fermentationslösung abzutrennen, um Milchsäure höherer Qualität (Pharmakopöe-Milchsäure, Plastik-Milchsäure) zu erzeugen.

Eine Möglichkeit, Milchsäure von Pharma-Qualität herzustellen, ist die Wasserdampfdestillation mit überhitztem Heißdampf unter Vakuum. Milchsäure zeigt bekanntlich mit Wasserdampf bei 100° C eine sehr geringe Flüchtigkeit. Die Wasserdampfflüchtigkeit kann jedoch erheblich gesteigert werden, wenn man überhitzten Dampf im Temperaturbereich zwischen 160 - 200° C verwendet. Basierend auf diesem Befund wurden Reinigungsverfahren für Milchsäure durch Wasserdampfdestillation ausgearbeitet, die z.B. in den Patenten DK 83589 (1957) oder CS 97136 (1960) ihren Niederschlag fanden. Dieses mit Abstand älteste Verfahren zur Herstellung von Pharmakopöe-Milchsäure hat sich in der Praxis aber nicht durchsetzen können, da wegen der relativen "Nichtflüchtigkeit" der Milchsäure diese Reinigungsmethode viel zu teuer kommt.

Mehr Erfolg hatte allerdings die Flüssig/Flüssig-Extraktion der Milchsäure mit org. Lösungsmitteln. Bei diesem Verfahren wird im Prinzip so vorgegangen, daß die von der Biomasse befreite Fermentationslösung mit Schwefelsäure angesäuert, der ausfallende Gips abfiltriert und schließlich mit Aktivkohle entfärbt und durch Ionenaustausch entsalzt wird. Die so hergestellte Rohmilchsäurelösung wird dann im Vakuum auf eine bestimmte Konzentration eingeengt und in einer Gegenstromextraktionskolonne mit einem org. Lösungsmittel in Kontakt gebracht. Aus der org. Phase kann die Milchsäure dann entweder durch Rückextraktion mit Wasser oder durch Aodestillieren des org. Lösungsmittel gewonnen werden. Häufig ist nach dem Extraktionsprozeß noch eine Nachbehandlung der Reinmilchsäurelösung mit Aktivkohle und Ionenaustauschern erforderlich, ehe sie auf die übliche Handelskonzentration von meist 80 % aufkonzentriert werden kann.

Ein derartiges Verfahren wird z.B. von JENEMANN (1933) im US-Patent 1906068 beschrieben, wobei als Lösungsmittel Iso-Propyl-Äther verwendet wird.

Ein Extraktionsverfahren, das als org. Phase Nitroparaffin verwendet, wird von TINDALL (1940) im US-Patent 2223797 vorgeschlagen.

Auch in jüngerer Zeit hat es nicht an Versuchen gefehlt, den Gewinnungsprozeß für Milchsäure durch Extraktion zu verbessern.

So wird z.B. in der DE-OS 3415141 ein Extraktionsverfahren vorgestellt, bei dem Butanole oder Pentanole als Lösungsmittel verwendet werden. Die Besonderheit bei diesen Verfahren liegt darin, daß die Ca-Lactat enthaltende Brühe sofort nach Beendigung der Fermentation mit Schwefelsäure angesäuert und die erhaltene Suspension, die als Feststoffe Gips und Biomasse enthält, direkt in einer mit hydrophoben Einbauten (z.B. aus Teflon) ausgerüsteten pulsierten Gegenstromkolonne mit dem Lösungsmittel in Berührung gebracht wird. Nach der Extraktion der wäßrigen Suspension durch das Lösungsmittel, die man bevorzugt bei einer Temperatur von 70° C durchführt, entnimmt man der Kolonne eine feststoffhaltige wäßrige Phase und eine feststofffreie org. Phase. Die in der org. Phase gelöste Milchsäure wird schließlich durch Abdestillieren des Reaktionswassers bei 60° - 140° C (gegebenenfalls unter Vakuum) vollständig in den Milchsäureester überführt. Dieser kann durch Destillation im Vakuum in reiner Form erhalten werden und stellt ein wertvolles Zwischenprodukt da. Die Ester können in bekannter Weise wieder zu Milchsäure und Alkohol gespalten werden, so daß auf diese Weise hochreine Milchsäure pharmazeutischer Qualität erhalten wird.

Ein großer Nachteil aller Extraktionsmethoden liegt darin, daß die meisten der verwendeten org. Lösungsmittel für Milchsäure nur eine sehr geringen Verteilungskoeffizienten haben, wodurch sehr große Mengen an organischen Lösungsmitteln erforderlich sind.

Der Verteilungskoeffizient für Milchsäure läßt sich allerdings wesentlich verbessern, wenn für die Extraktion eine Mischung aus org. Lösungsmitteln mit einem tertiären Amin verwendet werden. Ein auf dieser Grundlage beruhendes Reinigungsverfahren wird z.B. im US-Patent 4698303 (1987) beschrieben, wobei als Extraktionsmittel vorallem eine Mischung aus etwa 60 - 75 % Isobutylheptylketon und 25 - 40 % Adogen 364 als besonders effizient erwiesen hat. Adogen 364 ist der Handesname (Sherex Co.) einer Mischung langkettiger (C 8 - C 10) tertiärer Amine.

Bei diesen Reinigungsverfahren bereitet aber die Rückgewinnung der Milchsäure aus der org. Phase gewisse Schwierigkeiten, da sie nur durch Rückextraktion mit einer basischen Lösung (bevorzugt wird Ammoniumhydroxyd verwendet) gelingt. Dies bedeutet, daß nach der Extraktion noch weitere Reinigungsschritte erforderlich sind.

Die durch Flüssig/Flüssig-Extraktion gewonnene Milchsäure ist zwar im wesentlichen aschefrei, enthält allerdings noch andere, aus dem Rohmaterial stammende Verunreinigungen. Um mit dieser Methode Milchsäure pharmazeutischer Qualität zu erhalten, bedarf es einerseits sehr reiner Ausgangsmaischen, sowie andererseits noch zusätzlicher Behandlungen, z.B. mit Aktivkohle, Oxidationsmittel und Ionenaustauschern. (VICKROY, T.B., Lactic Acid in. Comprehensive Biotechnology, Vol 3, 761-776, Pergamon Press) (PECKHAM, G.T., 1944, Chem. Eng. News, 22, 440-443).

Die am häufigsten angewandte Methode zur Herstellung von Pharmakopöe-Milchsäure ist daher die Veresterung der Milchsäure mit niedrigen Alkoholen (meist Methanol) und der anschließenden Abtrennung der Ester durch fraktionierte Destillation. In der Literatur sind zahlreiche Methoden zur Reinigung von Milchsäure mittels Veresterung beschrieben worden. Bei einem Teil dieser Verfahren wird die, auf eine bestimmte Konzentration eingeengte Rohmilchsäure, der man im allgemeinen einen sauren Katalysator zusetzt, der Einwirkung von Alkoholdämpfen unterworfen. Mit dem entweichenden Dampfgemisch wird Milchsäure größtenteils als Ester von den Begleitsubstanzen abgetrennt. In einer anschließenden Rektifikationskolonne wird der überschüssige Alkohol abgetrennt und zurückgeführt. Das Methyllactat kann letztlich mit Wasser wieder zu Methanol und Milchsäure hydrolysiert werden.

Die Veresterungsreaktion von konzentrierter Milchsäure mit Methanol in Gegenwart eines sauren Katalysators, stellt hinsichtlich der Reinigung keine Schwierigkeiten dar. So wird z.B. in der DE-OS 1912730 ein Verfahren zur Herstellung von Milchsäuremethylester in Gegenwart eines sauren Ionenaustauschers beschrieben, wobei der Ester durch fraktionierte Vakuumdestillation in 82 %iger Ausbeute erhalten wird.

Schwieriger wird die Reinigung der Ester, wenn man von einer verdünnten wässrigen Milchsäurelösung ausgeht, weil in Gegenwart von Wasser der Ester sehr leicht wieder gespalten werden kann. Gemäß der US-PS 2350370 wird zwar verdünnte, wäßrige Milchsäure mit saurem Katalysator verestert, jedoch wird der abdestillierte Ester gleich wieder verseift, um auf diesem Wege Milchsäure zu reinigen.

Im DE-OS 3214697 wird ein Verfahren zur kontinuierlichen Reinigung von Milchsäuremethylestern vorgeschlagen, bei dem der durch säurekatalysierte Veresterung einer verdünnten Milchsäure hergestellte Ester zuerst durch Teilkondensation des bei der Veresterung entstehenden Gasgemisches und einer anschließenden Vakuumdestillation angereichert, und der im Sumpf der 1. Trennkolonne verbliebene Rohmilchsäuremethylester, der im wesentlichen nur noch geringe Mengen an Milchsäure enthält, zur vollständigen Reinigung in eine zweite Trennkolonne geleitet wird.

Wenngleich das "Veresterungs-Verfahren" die zur Zeit einzige brauchbare Methode zur Herstellung von Pharmakopöe-Milchsäure ist, weist sie doch den Nachteil auf, daß auch hier mit großen Mengen organischer Lösungsmittel gearbeitet werden muß, was ein erhebliches Sicherheits- und Umweltrisiko darstellen.

Es wurde daher verstärkt nach alternativen Verfahren gesucht, die die oben erwähnten Nachteile nicht aufweisen.

So sind eine Reihe von Patentschriften bekannt, bei denen die Abtrennung der org. Säuren durch Elektrodialyse vorgeschlagen wird. Im AT 290441 wird z.B. ein Verfahren zur Reinigung von Milchsäure vorgestellt, bei dem durch eine Kombination von Elektrodialyse und Extraktion eine Milchsäure erhalten wird, die frei von "unangenehmen Geruch und Geschmack" sein soll. Bei diesem Verfahren wird eine auf ca. 20 % aufkonzentrierte Rohmilchsäurelösung einer Elektrodialysebehandlung unterzogen und die dialysierte Lösung anschließend mit einem org. Lösungsmittel (empfohlen wird Isopropyläther) extrahiert. Aus der org. Phase wird die Milchsäure durch Rückextraktion mit Wasser gewonnen.

Im EP 0393818 wird ein Verfahren zur Reinigung von Milchsäure vorgeschlagen, bei dem das in der Fermentationsbrühe enthaltene Milchsäure-Salz (z.B. $NH_4$-Lactat) zuerst durch konventionelle Elektodialyse abgetrennt wird. Das so gewonnene Milchsäure-Salz wird anschließend in einen zweiten Elektrodialysator geleitet, der mit bipolaren Membranen ausgestattet ist, und in dem durch Wasserspaltung das Milchsäure-Salz in die freie Säure und deren korrespondierende Base zerlegt wird. Letztlich wird die Milchsäurelösung, zur Entfernung der noch vorhandenen An- und Kationen, über ein stark basisches und eine stark saures Ionenaustauscher-Harz geleitet. Auf diese Weise soll eine hochgereinigte Milchsäure erhalten werden.

Die mit Elektrodialyse arbeitenden Verfahren haben allerdings den Nachteil, daß sie einerseits große Mengen an elektrischer Energie benötigen, was das Verfahren sehr verteuert und andererseits zur Herstellung hochreiner Milchsäure noch weitere Reinigungsschritte, wie z.B. Ionenaustausch, erforderlich sind.

Ein ebenfalls alternatives Verfahren zur Gewinnung und/oder Reinigung fermentativ hergestellter Carbonsäuren ist das Ionenaustauschverfahren an sauren und/oder basischen Ionenaustauscher-Harzen.

So geht aus der JP-A-62238232 hervor, Ammoniumverbindungen von Carbonsäuren durch Absorption des Ammoniumkations an einem Kationentauschharz, insbesondere an einem schwach sauren Kationentauscherharz und Desorbtion und Wiedergewinnung der Ammoniumkationen als Ammoniumion mittels eines organischen Lösungsmittels umzuwandeln. Die entsprechend gebildeten Carbonsäuren können dann in Form einer wässrigen Lösung gewonnen werden.

Aus der Veröffentlichung im Chemical Abstract, Band 78 (1973), 135585b, geht hervor, Milchsäure, die durch Fermentation von Zucker gewonnen wurde, mit Kationenaustauschern zu reinigen, wobei insbesondere Kupfer-, Eisen-, Zink- und Bleigehalte auf etwa 1, 2, 4 und 0 mg/kg reduziert wurden.

So wird z.B. in DD-PS 203533 ein Ionenaustauschverfahren zur Gewinnung von Carbon- und Oxycarbonsäuren aus deren fremdsalzhaltigen Lösungen beschrieben, bei dem die Salze zuerst über stark saure Kationenaustauscher in der $H^+$-Form in Säuren umgewandelt werden. Dabei wird ein Gemisch aus Carbonsäuren und Fremdsäuren erhalten. Mit einer geringer konzentrierten Fraktion dieses Gemisches wird sodann ein vorerst in der Basen- oder Hydroxylform vorliegender schwach basischer Anionenaustauscher beladen, also im wesentlichen in die Carbonsäureform überführt. Anschließend wird über den säurebeladenen Ionenaustauscher eine höher konzentrierte Fraktion des in der Entkationisierungsstufe erhaltenen Säuregemisches geleitet, wobei die zu gewinnende Säure das Harzbett ungehindert passiert und lediglich die stärkeren Fremdanionen (erwähnt ist Chlorid) durch den Ionentausch an das Harz gebunden werden.

Im EP 0135728 wird ein Verfahren zur "Isolierung von enzymatisch erzeugten Carbonsäuren" beschrieben, bei dem die, vorteilhaft durch kontinuierlich Fermentation hergestellte Lösung über einen "Desorber" läuft, der mit einem "Polymerisat mit tertiären Aminogruppen" gefüllt ist, welcher selektiv Carbonsäuren adsorbiert. Die aus dem "Desorber" austretende weitgehend carbonsäurefreie Flüssigkeit wird wieder in den Reaktor zurückgeführt. Nach Beladen eines "Desorbers" wird die Reaktionslösung auf den nächsten Desorber geführt und aus dem beladenen Desorber die Carbonsäure mit Hilfe eines polaren Lösungsmittel, z.B. mit Methanol, eluiert. Die Abtrennung der Carbonsäure aus dem Eluat erfolgt nach bekannten Methoden; im Falle flüchtiger Elutionsmittel z.B. durch Destillation.

Weitere Verfahren zur Abtrennung von Milchsäure durch Ionenaustausch werden im US-PS 3202705 und im JP 91183487 vorgeschlagen. Bei diesen Verfahren wird die mit Schwefeläure angesäuerte Fermentationsbrühe nach

Abtrennung des ausfallenden $CaSO_4$ zuerst über einen stark sauren Kationenaustauscher in der $H^+$-Form und anschließend über einen basischen Anionenaustauscher geleitet. Auf diese Weise soll, zumindest im Falle des US-Patens, eine "farbstabile" Milchsäure erhalten werden.

Der große Nachteil aller Verfahren, bei denen ein "echter" Ionenaustausch stattfindet, ist die erforderliche, teure Regeneration der Harze.

Als jüngste alternative Verfahren zur Gewinnung und Reinigung org. Säuren werden deshalb Chromatographieverfahren an basischen Anionenaustauschern beschrieben.

So wird im EP 0324210 ein Reinigungsverfahren vorgestellt, bei dem eine fermentativ hergestellte Zitronensäure durch Adsorption an neutrale, nichtionogene, makroretikulare, wasserunlösliche Harze bzw. an schwach oder stark basischen Anionenaustauschern, gereinigt wird. Als Eluens wird Wasser, eine Wasser-Acetonmischung oder eine verdünnte Schwefelsäurelösung verwendet. Dieses Verfahren ist in der Lage, Salze und Kohlenhydrate von der Zitronensäure abzutrennen.

Nach genau dem selben Prinzip arbeitet das im EP-OS 0377430 vorgeschlagene Verfahren. Bei diesem werden zur chromatographischen Isolierung und/oder Reinigung von Säuren ebenfalls basische Ionenaustauscher vorgeschagen. Der Unterschied zum EP 0 324 210 besteht vor allem darin, daß nicht nur Zitronensäure, sondern auch andere anorganische (z.B. Phosphorsäure) und organische (z.B. Weinsäure, Äpfelsäure oder Milchsäure) Säuren auf diese Weise getrennt und/oder gereinigt werden können.

Alle IAC-Methoden, die basische Anionenaustauscher oder nichtionogen Adsorberharze verwenden, haben allerdings den großen Nachteil, daß die auf den Harz adsorptiv zurückgehaltenen Säuren bei ihrer Elution mit Wasser oder verd. Schwefelsäure ein starkes "Tailing" aufweisen, was zu einer starken Verdünnung der eluierten Säure führt. Darüber hinaus ist es mit dieser Methode nicht möglich, Säuren mit ähnlichen pKs-Werten, wie z.B. Milchsäure und Essigsäure voneinander zu trennen.

Der Erfindung liegt die Aufgabe zugrunde, Milchsäure aus salz- und kohlenhydrathältigen Substraten (Fermentationsmaischen) auf einfache Weise und zuverlässig zu gewinnen.

Diese Aufgabe wird erfindungsgemäß dadurch gelöst, daß der Abtrennungs- und Reinigungsprozeß in zwei Schritten vorgenommen wird, wobei

a) im ersten Schritt, in einer oder mehreren "Vorsäulen", die in der Fermentationslösung gegebenenfalls vorhandenen Salze, vor allem das Salz der Milchsäure, durch einen echten Ionenaustausch in die freien Säuren umgewandelt werden und

b) im zweiten Schritt, in einer oder mehrerer "Trennsäulen", die freie Milchsäure durch Chromatographie an stark sauren Ionenaustauschern von den übrigen in der Fermentationslösung vorhandenen Säuren, Kohlenhydraten und sonstigen Verunreinigungen getrennt wird.

Durch dieses Verfahren ergeben sich folgende Vorteile:

- Die Fermentationsmaische muß zur Freisetzung der Milchsäure nicht mit einer starken Mineralsäure versetzt werden, was die Salzfracht der Maische erheblich verringert. Der Vorteil gegenüber den Verfahren, bei denen die Milchsäure durch klassischen Ionenaustausch aus der meist angesäuerten Brühe mit Hilfe stark saurer Kationenaustauscher abgetrennt wird, liegt vor allem darin, daß zur Regeneration eines schwach sauren Ionenaustauscher um etwa 2/3 weniger Mineralsäure benötigt wird.
- Der Vorteil gegenüber den IAC-Verfahren mit basischen Anionenaustauschern besteht darin, daß die Milchsäurefraktion als scharfer, symmetrischer Peak ohne "Tailing" erhalten wird.
- Weiters ist am stark sauren Ionenaustauscher auch die Trennung der Milchsäure von anderen vorhandenen, organischen Säuren, insbesonders der Essigsäure, möglich. Dadurch kann Milchsäure von pharmazeutischer Qualität und ausreichender Farbstabilität erhalten werden, so daß sie auch für Polymerisationszwecke eingesetzt werden kann.
- Als Elutionsmittel kann beim gegenständlichen Verfahren reines Wasser benutzt werden, im Gegensatz zu den IAC-Verfahren mit basischen Austauschern, die verdünnte Sauren zur Elution benötigen.

Die Fermentationslösung, aus welcher die Milchsäure abgetrennt werden soll, muß einen pH-Wert über 5,0 aufweisen und somit noch im Arbeitsbereich schwach saurer Kationenaustauscher liegen.

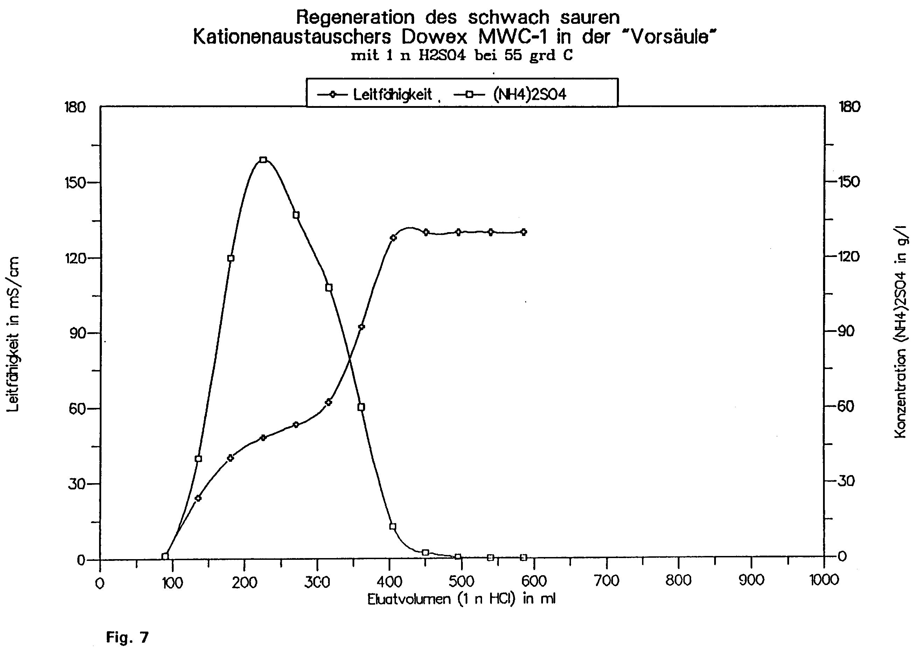

Das in einer oder in mehreren "Vorsäulen" befindliche Harz ist ein Kationenaustauscher, vorzugsweise ein schwach saurer Kationenaustauscher in der $H^+$-Form, da bei diesem die Regeneration in die $H^+$-Form praktisch mit der theoretischen Säuremenge möglich ist (Fig. 7).

Die Temperatur der "Vorsäule" soll mindestens 50°C, vorzugsweise jedoch 70° - 80°C betragen, weil dadurch die Acidität und somit auch das Salzspaltungsvermögen schwach saurer Kationenaustauscher erhöht und die nutzbare Kapazität (Durchbruchskapazität) des Austauschers wesentlich verbessert wird.

Der in einer oder in mehreren "Trennsäulen" befindliche stark saure Kationenaustauscher liegt in der $H^+$-Form vor,

EP 0 684 941 B1

wodurch eine chromatographische Trennung der Milchsäure auch von anderen organischen Säuren, insbesondere der Essigsäure, möglich ist.

Die entkationisierte Fermentationslösung wird durch den Kontakt am stark sauren Kationenaustauscher in eine, die nichtmilchsäurehältigen Bestandteile enthaltende Raffinatfraktion I (Vorlauf), eine milchsäurehältige Produktfraktion und eine vornehmlich essigsäurehältige Raffinatfraktion II (Nachlauf) aufgetrennt.

Als Elutionsmittel zum Herauswaschen der einzelnen Fraktionen aus den Trennsäulen wird reines, vorzugsweise entionisiertes Wasser verwendet.

Die Elutionstemperatur liegt zwischen der Raumtemperatur und der Stabilitätsgrenztemperatur der verwendeten Harze, vorzugsweise bei 50°-65°C, wodurch einerseits eine mikrobielle Infektion verhindert und andererseits die Anzahl der theoretischen Böden erhöht wird.

Es wird nur der in den "Vorsäulen" befindliche schwach saure Kationenaustauscher nach seiner vollkommenen Beladung mit den Kationen der Fermentationslösung mit einer verdünnten starken Mineralsäure, vorzugsweise mit einer 1 - 2 n Schwefelsäure regeneriert.

Die bei der Regeneration der Harze in den Vorsäulen anfallende verdünnte Salzlösung wird durch salzspaltende Elektrodialyse in die korrespendierenden Säuren und Basen gespalten und wieder in den Prozeß zurückgeführt.

Im einzelnen gestaltet sich die Durchführung des erfindungsgemäßen Verfahrens in der Weise, daß eine bevorzugt durch Verwendung von Ammoniumwasser als Neutralisationsmittel, durch Fermentation mit Lactobacillus delbrückii hergestellte Milchsäuremaische von grobdispersen Bestandteilen (Biomasse) befreit wird. Das geschieht zum Beispiel durch Zentrifugation mit einer Vollmantelzentrifuge. Zur Entfernung von Farbstoffen und lipophilen Bestandteile kann die Fermentationslösung mit Aktivkohle versetzt werden. Nach einer Kontaktzeit wird letztere in der üblichen Weise durch Filtration entfernt. Die Maische wird gegebenenfalls auf 30-50 % (G/G) durch Vakuumverdampfung aufkonzentriert und stellt die im folgendem als "Feed" bezeichnete Lösung dar.

Der eigentliche, erfindungsgemäße Reinigungs- und Trennprozeß der Milchsäure erfolgt in zwei Schritten:

1) durch einen echten Ionenaustausch an einem bevorzugt schwach sauren Kationenaustauscher in der $H^+$-Form, und
2) durch einen IA-Cromatographieprozeß an einem stark sauren Kationenaustauscher in der $H^+$-Form.

Der Prozeß beginnt damit, daß eine bestimmtes Volumen der Fermentationslösung ("Feed") auf die "Vorsäule" aufgebracht wird. Die pro Chromatographiezyklus aufzubringende Menge an "Feed"-Lösung hängt einerseits von deren Konzentration sowie andererseits vom Durchmesser der "Trennsäulen" ab. Sie wird aber im allgemeinen zwischen 5 - 10 % des Harz-Bettvolumen der "Trennsäule" betragen.

Ist die "Feed"-Lösung zur Ganze in die "Vorsäule" eingedrungen, wird sofort auf reines, vorzugsweise entionisiertes Wasser umgeschalten, welches als Elutionsmittel in diesem Prozeß verwendet wird.

In der "Vorsäule" befindet sich ein bevorzugt schwach saurer Kationenaustauscher in der $H^+$-Form, durch welchen die in der "Feed"-Lösung enthaltenen Salze, vor allem das Salz der Milchsäure ($NH_4$-Lactat), durch Ionenaustausch in die entsprechenden Säuren umgewandelt werden. Das Harz-Bettvolumen der "Vorsäule" muß nur so groß sein, daß bei den gegebenen Betriebsbedingungen die sog. "Durchbruchskapazität" des in der Vorsäule verwendeten Harzes ausreicht, die pro Chromatographiezyklus aufgebrachte Menge an Kationen gegen $H^+$-Ionen auszutauschen. Da die "Durchbruchskapazität" schwach saurer Austauscher, neben den pH der "Feed"-Lösung vor allem von der Betriebstemperatur abhängt, ist es vorteilhaft, den Ionenaustausch-Prozeß in der "Vorsäule" bei mindestens 50° C, bevorzugt jedoch bei 70 - 80° C ablaufen zu lassen.

Die von der "Vorsäule" kommende, entkationisierte "Feed"-Lösung wird dann mit Hilfe einer Pumpe direkt auf die "Trennsäule" befördert, die mit einem stark sauren Kationenaustauscher in der $H^+$-Form gefüllt ist. Mit der Pumpe wird auch die für den Chromatographie-Prozeß erforderliche, optimale Durchflußgeschwindigkeit konstant gehalten. In der "Trennsäule" findet der eigentliche IAC-Prozeß statt, der zur Trennung der Milchsäure von den übrigen, in der "Feed"-Lösung vorhandenen Komponenten führt. Der aus der "Trennsäule" ausfließende Eluatstrom kann in drei Fraktionen geteilt werden:

*) in die Raffinatfraktion I, (Vorlauf) welche die nichtmilchsäure Komponenten der "Feed"-Lösung enthält (Kohlenhydrate, starke Säuren, Proteine)
*) in die Protuktfraktion, welche die freie Milchsäure enthält und
*) in die Raffinatfraktion II, (Nachlauf) welche in erster Linie Essigsäure enthält.

Der IAC-Prozeß in den "Trennsäulen" findet vorzugsweise bei einer Temperatur von 50° - 65° C statt. Dadurch wird einerseits eine mikrobielle Infektionen des Harzes verhindert und andererseits die Anzahl der theoretischen Böden erhöht.

Der in der "Vorsäule" befindliche, schwach saure Kationenaustauscher wird nach seiner Beladung mit den Kationen der "Feed"-Lösung, mit einer starken Mineralsäure (z.B. mit einer 1 - 2 n Schwefelsäure), im Gegenstromverfahren, regeneriert. Die bei der Regeneration der "Vorsäule" anfallende, verdünnte Salzlösung, die zum überwiegenden Teil aus Ammoniumsulfat besteht, kann durch satzspaltende Elektrodialyse in die korrespondierenden Säuren (Schwefelsäure) und Basen (Ammoniumhydroxid) zerlegt und wieder in den Prozeß zurückgeführt werden.

Wie bereits oben erwähnt, muß während der Fermentation der Milchsäure, der für den Mikroorganismus optimale pH-Wert konstant gehalten werden, um eine befriedigende Produktivität zu erreichen. Dies geschieht in einfacher Weise dadurch, daß der Maische während der Fermentation ein Neutralisationsmittel, wie z.B. $NH_4OH$, zugegeben wird, wodurch der pH-Wert der Lösung auf 5,5 - 6,5 konstant gehalten werden kann. Die aus der Fermentation kommende Maische enthält also, neben geringen Mengen von aus dem Nährmedium stammenden Salzen, Kohlenhydrate und anderen Verunreinigungen, als Hauptkomponente das Salz der Milchsäure, also z.B. das $NH_4$-Lactat.

Bei der chromatographischen Trennung von Mehrkomponenten-Systemen, wie der oben beschriebenen Milchsäuremaische, kommen mehrere Effekte zum Tragen.

Als Erster dieser Effekte wäre der von WHEATON und BAUMANN (1953) beschriebene "Ionenausschlußeffekt" zu nennen. (Ing. Eng. Chem.; 45 (1953) 228) Er ermöglicht die Trennung ionischer von nichtionischen Komponenten. Im Gegensatz zum herkömmlichen Ionenaustauschverfahren findet hier kein Ionenaustausch statt, wodurch auch keine Regeneration des Harzes erforderlich wird. Theoretisch kann der Ionenausschlußeffekt durch die "Donnan-Membrantheorie" erklärt und beschrieben werden. (MEYER, W.R.S. et al.; Ind. Eng. Chem. Proc. Des. Develop; 6 (1967) 55). Wird z.B. eine Salzlösung $K^+A^-$ mit einem Kationenaustauscherharz, welches vorher mit dem Kation $K^+$ abgesättigt wurde, zusammengebracht, so nimmt das Harz eine negative Ladung gegenüber der Lösung an. Diese negative Ladung ist das Ergebnis einer geringfügigen Wanderung des Anions $A^-$ in das Harz und einer ähnlichen Wanderung des Kations $K^+$ in das Harz-Zwischenraumvolumen. Es bildet sich eine Potentialdifferenz zwischen Lösung und Harzphase aus. Die Größe dieser Potentialdifferenz kann durch das Donnanmembranpotential beschrieben werden.

Für die praktische Anwendung bedeutet dies, daß gelöste Salze, die als Kation dasselbe Ion enthalten wie das Gegenion des Harzes, nicht in das Harz eindringen können und als erste Komponente die Säule wieder verlassen . Dasselbe Prinzip gilt auch für Säuren wenn sie an einem stark sauren Kationenaustauscher in der $H^+$-Form getrennt werden sollen. Bei Säuren spielt allerdings noch der pKs-Wert der betreffenden Säure eine Rolle. Je niedriger der pKs der Säure (also je starker die Säure) ist, um so weniger wird die Säure vom Harz zurückgehalten, und umgekehrt. Dies bedeutet, daß starke Säuren auf grund des Donnan-Ausschlusses nicht in die Harzporen eindringen können, wärend mittelstarke und schwache Säuren, je nach ihrem Dissoziationsgrad, mehr oder weniger weit in die Harzporen eindringen können und daher erst später die Säule wieder verlassen werden.

Ein weitere Effekt der bei der IA-Cromatographie zum Tragen kommt, ist der sog. "Molekularsiebeffekt" der für die Auftrennung ungeladener (nichtionischer) gelöster Komponenten verantwortlich ist. (WALTER, H.G. et al.; Cer. Sci. Today; 15 (1970) 140) Die Porengröße eines Ionenaustauscherharzes wird durch seinen Vernetzungsgrad bestimmt. Ob nun ein Molekül beim Durchwandern einer Ionenaustauchersäule aus dem Zwischenraum- in das Porenvolumen gelangen kann, hängt von seiner Größe und Form bzw. eben von der Porengröße des Harzes ab. Der Ionenaustauscher wirkt wie ein Molekularsieb, welches Moleküle einer bestimmten Form und Größe aus dem Porenvolumen ausschließt. (NORMANN, L.G.; J.Amer. Soc. Sugar Beet Tech.; 12 (1963) 363) Jene Komponenten, die nicht in die Poren gelangen können, werden beim Eluieren der Säule früher im Eluat erscheinen als jene, die sich gleichmäßig verteilen.

Ein Maß dafür, wie stark eine Substanz von dem betreffenden Harz zurückgehalten wird, ist durch den sog. "Verteilungskoeffizienten" ($K_{di}$) gegeben. Er ist definiert als das Verhältnis der Konzentrationen des gelösten Stoffes (i) im Harzporenvolumen ($C_{hi}$) zur Konzentration des gelösten Stoffes (i) im Zwischenraumvolumen ($C_{zi}$):

$$K_{di} = C_{hi}/C_{zi}$$

Ganz allgemein hängt der Verteilungskoeffizient für eine bestimmte Verbindung von der Struktur dieser Verbindung und deren Konzentration, vom Typ und der ionischen Form des Harzes und schließlich auch von den anderen, in der Lösung vorhandenen Verbindungen ab.

Die chromatographische Abtrennung der Milchsäure aus einer Lösung die neben ionischen (Salze) auch nichtionische (Kohlenhydrate) Komponenten enthält, gelingt an einem stark sauren Kationenaustauscher durch das Zusammenwirken der beiden beschriebenen Effekte. Eine Voraussetzung dafür ist allerdings, daß es während des Chromatographieprozesses zu keiner Umladung des Harzes kommt, d.h. daß das Gegenion des Kationenaustauschers dasselbe sein muß, wie das mengenmäßig am stärksten vorhandene Kation der Lösung. Dieses wäre z.B. im Falle einer Neutralisation der Maische mit $NH_4OH$ das $NH_4^+$-Ion.

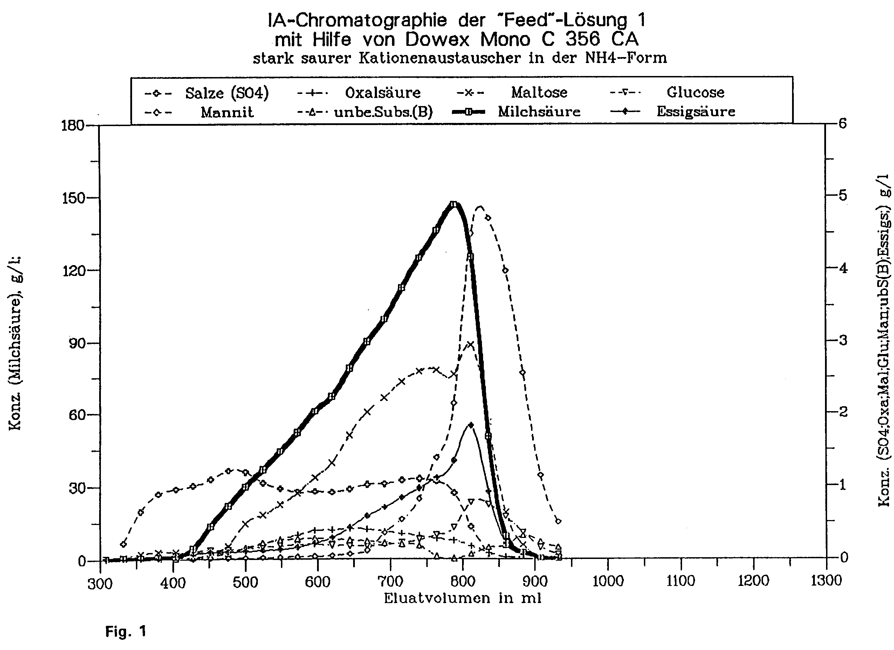

Rein theoretisch müßte also auf einem stark sauren Kationenaustauscher in der $NH_4^+$-Form eine chromatographische Trennung des $NH_4$-Lactats von den übrigen Komponenten der Maische möglich sein. Wie Versuche aber gezeigt haben, ist mit einem Kationenaustauscher in dieser Beladungsform eine Abtrennung des Milchsäuresalzes nicht oder nur sehr schwer möglich, da im Sinne des oben gesagten sich bei dieser Harzform die Verteilungskoeffizienten der einzelnen Komponenten zu wenig unterscheiden (Beispiel 1, Fig. 1).

Außerdem würde bei einem derartigen Prozeß nicht die freie Säure, sondern natürlich das Salz der Milchsäure,

also das $NH_4$-Lactat erhalten werden, welches in einen weiteren Schritt erst zur freien Säure umgewandelt werden müßte.

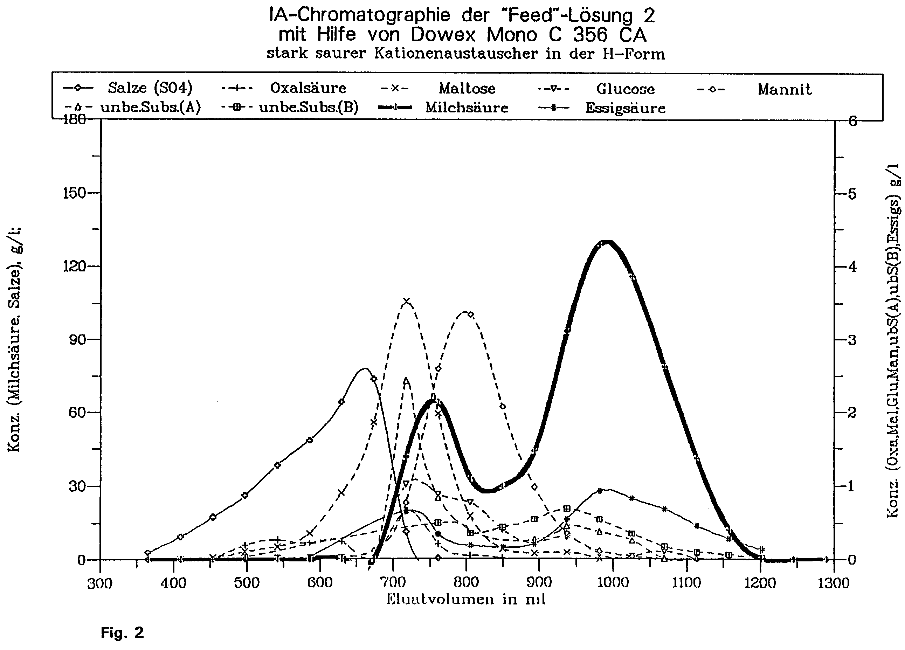

Führt man hingegen den IAC-Prozeß auf einem stark sauren Kationenaustauscher in der $H^+$-Form mit einer Maische durch, deren pH-Wert mit einer starken Mineralsäure (z.B. mit einer konz. $H_2SO_4$) auf 2,5 gesenkt wurde, ergibt sich ein ganz anderes Chromatogramm. Durch das Ansäuern der Maische werden die Säuren aus ihren Salzen gedrängt. In einer derartigen Lösung sind neben freier Milchsäure vor allem $(NH_4)_2SO_4$ und Schwefelsäure als Hauptkomponenten zu finden. Bei der chromatographischen Auftrennung dieser Lösung wird das mit dem "Feed" in das Trennharz eintretende Ammoniumsulfat zu einer teilweisen Umladung des Harzes führen. Die dabei entstehende Schwefelsäure wird mit der ohnedies im "Feed" vorhandenen Schwefelsäure, entsprechend dem "Ionenausschlußeffekt" die Trennsäule am schnellsten durchwandern und diese auch als erste Komponente verlassen. Die um vieles schwächer dissoziierte Milchsäure (pKs 3,86) wird hingegen langsamer durch die Trennsäule wandern und, ihrer Stärke entsprechend, einen Teil des zuerst in die $NH_4^+$-Form umgeladenen Harzes wieder in die $H^+$-Form zurückführen. Im Chromatogramm erscheint die Milchsäure als Doppelpeak, wobei der erste Peak das $NH_4$-Lactat und der zweite die freie Milchsäure darstellt (Beispiel 2, Fig. 2).

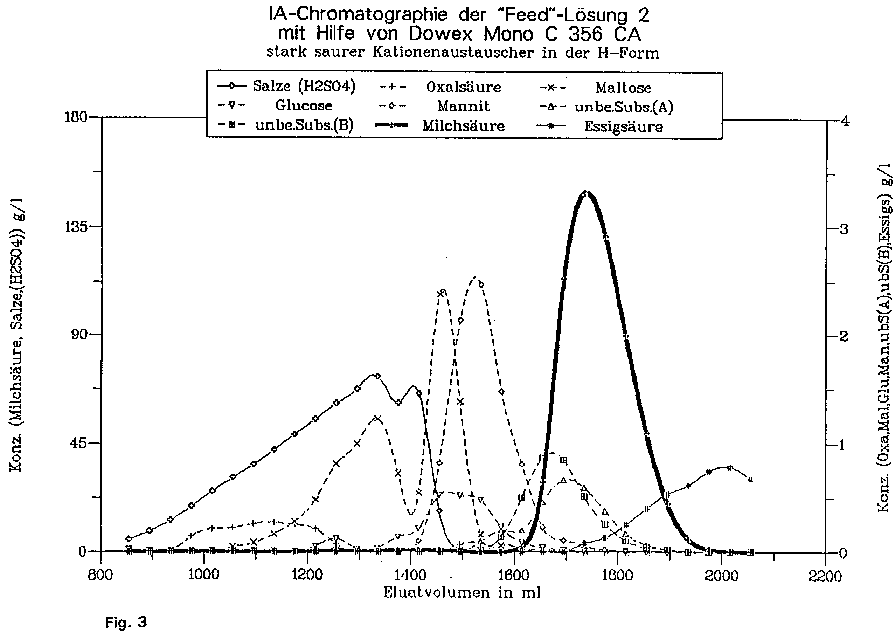

Der Doppelpeak, also die Aufteilung der im "Feed" vorhandenen Milchsäure in eine Salz- und eine Säurefraktion, kann jedoch vermieden werden, wenn nach dem Aufgeben der auf pH 2,5 angesäuerten "Feed"-Lösung, gleich oder in einem bestimmten Abstand, mit einer bestimmten Menge einer vorzugsweise 2 n $H_2SO_4$ nachgefahren wird. Die Menge der Schwefelsäure mit der nachgefahren werden muß, richtet sich nach der $NH_4$-Ionenkonzentration der "Feed"-Lösung. Nach der Schwefelsäure-Aufgabe wird wie üblich mit entionisiertem Wasser eluiert. Im Chromatogramm erscheint die freie Milchsäure als scharfer Einzelpeak. Der Grund dafür ist wieder in der Tatsache zu sehen, daß die starke Schwefelsäure um vieles schneller durch das Trennharz wandert als die schwächere Milchsäure oder gar als die noch schwächere Essigsäure. Die der "Feed"-Lösung nachgeschickte Schwefelsäure wird sozusagen die Milchsäure und anderen schwachen Säuren "überholen" und die vom Ammoniumsulfat verursachte Umladung des Harzes in die $NH_4^+$-Form wieder rückgängig machen (Beispiel 3, Fig. 3).

Bei diesem Prozeß findet also bereits eine Kombination von Ionenaustausch und IA-Chromatographie statt, wenngleich auf derselben Säule und am selben Harz.

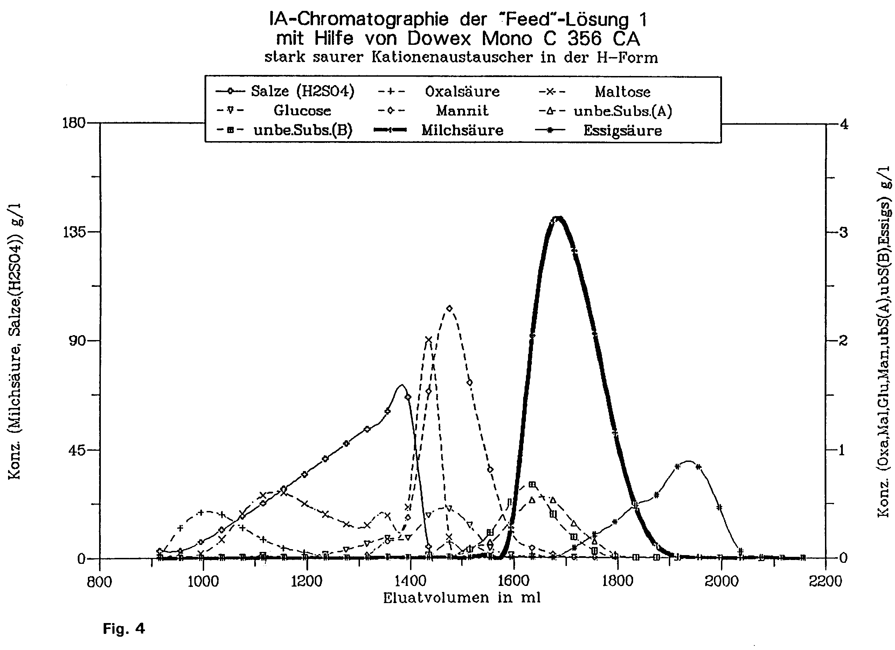

Die Verdrängung der Milchsäure aus ihrem Salz kann aber auch direkt durch Ionenaustausch auf dem Trennharz erfolgen. Wird als "Feed" eine nicht angesäuerte Maische (pH 5.8) auf einen stark sauren Kationenaustauscher in der $H^+$-Form aufgebracht und in der selben Weise wie oben geschildert mit einer bestimmten Menge Schwefelsäure nachgefahren und mit entionisierten Wasser eluiert, so ergibt sich das selbe Chromatogramm (Beispiel 4, Fig. 4).

Das oben geschilderte Verfahren, bei dem die beiden Prozesse "Ionenaustausch" und "IA-Chromatographie" auf ein und dem selben Harz, einem stark sauren Kationenaustauscher in der $H^+$-Form stattfinden, hat jedoch einen entscheidenden Nachteil. Dieser besteht darin, daß die in der Praxis mögliche Menge an Schwefelsäure, mit der der "Feed"-Lösung nachgefahren werden muß, nicht ausreicht um den Kationenaustauscher vollständig zu regenerieren. Es bleibt bei jedem Zyklus eine bestimmte Menge an $NH_4$-Ionen am Austauscher zurück, was bedeutet, daß nach einer bestimmten Anzähl von IAC-Zyklen der Austauscher vollständig regeneriert werden muß.

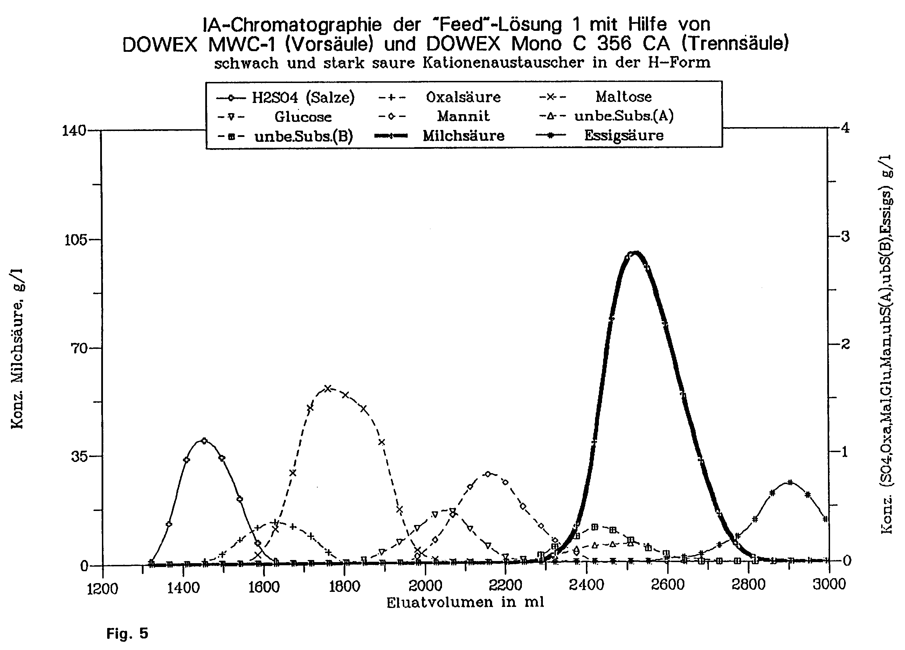

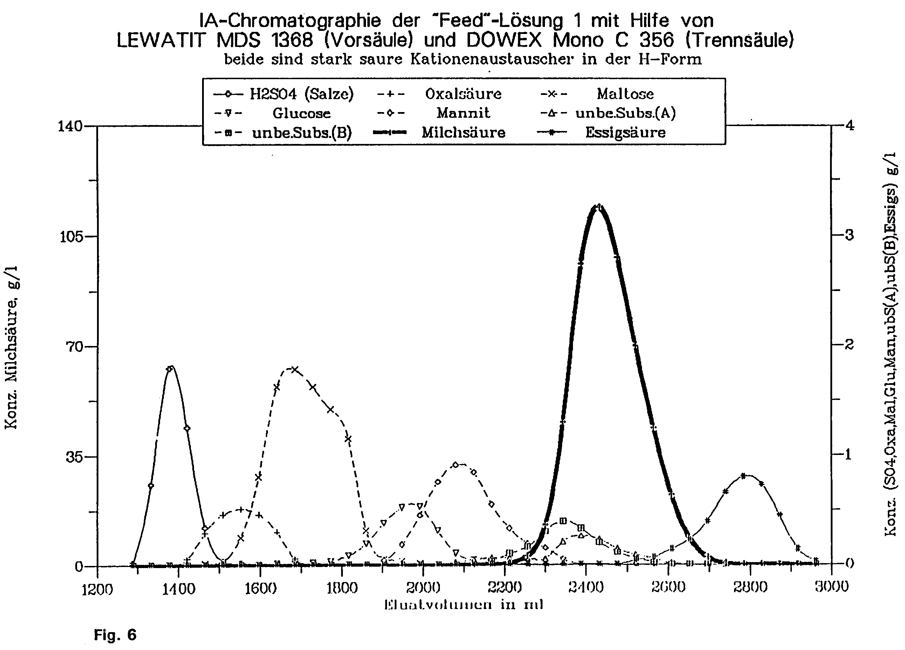

Dieser Nachteil kann auf einfache Weise dadurch beseitigt werden, indem man die beiden Prozesse, "Ionenaustausch" und "IA-Chromatographie", voneinander getrennt ablaufen läßt. Dazu ist es notwendig, der "Trennsäule" die mit dem stark sauren Kationenaustauscher in der $H^+$-Form gefüllt ist, und in der nur noch der IAC-Prozeß sattfinden soll, eine "Vorsäule" vorzuschalten, in der durch Ionenaustausch die Kationen die "Feed"-Lösung gegen $H^+$-Ionen ausgetauscht werden. Diese "Vorsäule" wird daher ebenfalls mit einem Kationenaustauscher in der $H^+$-Form gefüllt; dieser kann sowohl stark als auch schwach saurer Natur sein. Da der pH-Wert der Fermentationsmaische jedoch über 5, und somit noch im Arbeitsbereich schwach saurer Kationenaustauscher liegt bzw. ihre Acidität ausreicht um zumindest das Salz einer schwachen Säure (Milchsäure) zu spalten, ist es beim erfindungsgemäßen Verfahren von Vorteil, in der "Vorsäule" schwach saure Kationenaustauscher zu verwenden. Das Harz-Bettvolumen der "Vorsäule" hängt einerseits von der pro Chromatographiezyklus aufgetragenen "Feed"-Menge und andererseits von der "Durchbruchskapazität" des Harzes ab. Wenngleich schwach saure Kationenaustauscher eine sehr hohe Totalkapazität (bis zu 4 val/l Harz) aufweisen, wird ihre nutzbare Kapazität (Durchbruchskapazität) durch die Betriebsbedingungen verringert werden. Den größten Einfluß auf die nutzbare Kapazität schwach saurer Kationenaustauscher hat, neben den pH-Wert der Lösung (soweit dieser unter 7 bzw. im Bereich zwischen 7 - 4 liegt), vorallem die Betriebstemperatur. So nimmt die Acidität und damit das Salzspaltungsvermögen des Carbonsäureharzes auch gegenüber Neutralsalzen mit steigender Temperatur zunächst nur wenig, oberhalb von 50° C jedoch sehr stark zu. Es ist daher erforderlich, die "Vorsäule" bei einer möglichst hohen Temperatur, vorzugsweise bei 70 - 80 ° C zu betreiben (Beispiel 5 und 6, Fig. 5 und 6).

Wie den beiden Abb. 5 und 6 entnommen werden kann, ist bereits mit der in der Versuchsanlage zur Verfügung stehenden Trennstrecke von ca. 800 cm, eine weitgehende Abtrennung der Milchsäure erreichbar. Mit den in der Praxis üblichen Trennstrecken von 15 - 20 m ist, nach dem Gesetz der "theoretischen Böden", jedoch eine vollkommene Abtrennung der Milchsäure zu erwarten. Vor allem gelingt es, die Kohlenhydrate vollkommen von der Milchsäure abzutrennen, wie der in Beispiel 5 gezeigte "Hitze"-Test beweist, bei dem es nach 1-stündiger Erhitzung der milchsäurehaltigen Fraktion auf 200° C zu keiner Verfärbung kam.

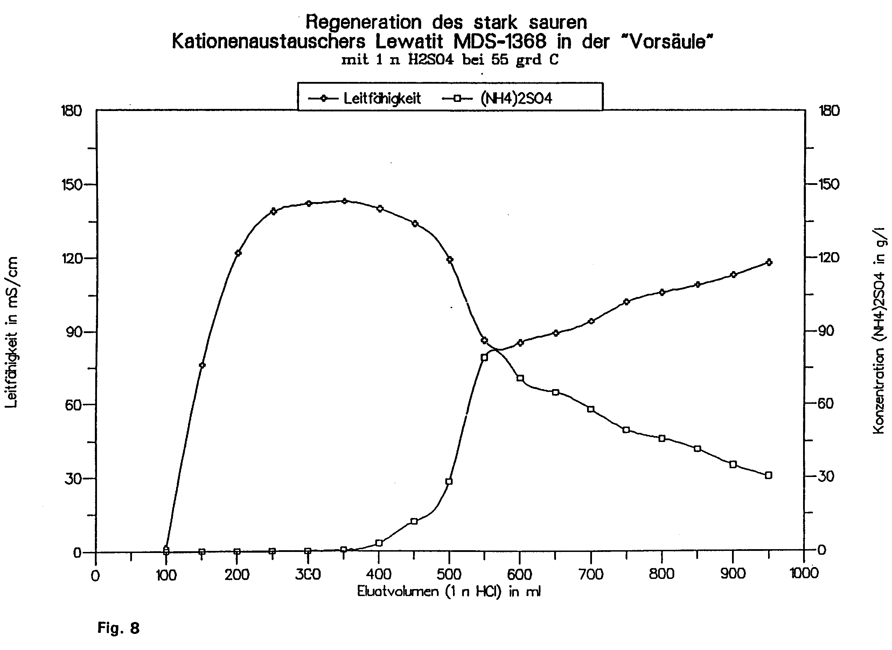

Ein entscheidender Vorteil schwach saurer Kationenaustauscher gegenüber stark sauren liegt in der hohen Chemikalienausnutzung bei der Regeneration. Wegen der niedrigen Dissoziationskonstante ist ihre Regeneration in die $H^+$-Form praktisch mit der theoretischen Säuremenge möglich (Beispiel 7 und 8, Fig. 7 und 8).

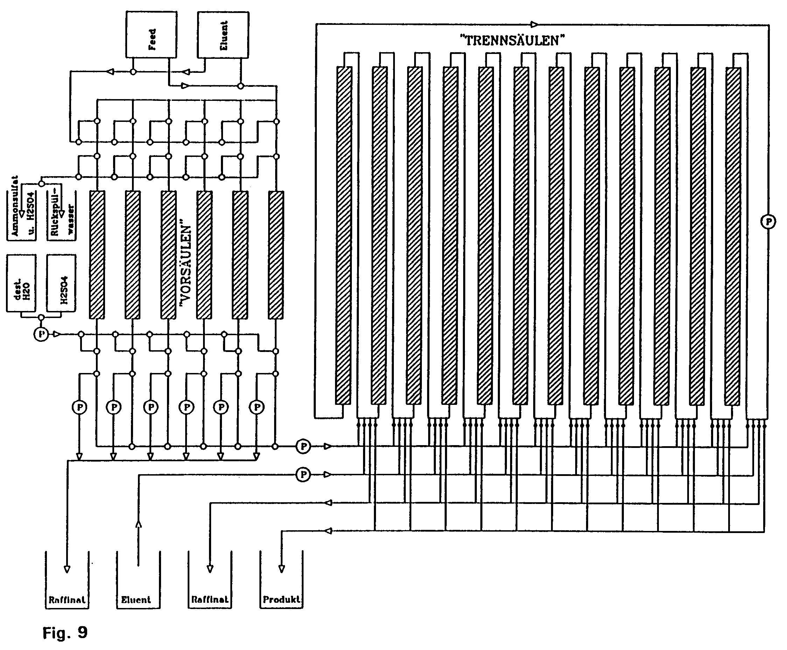

Das oben beschriebene Verfahren kann sowohl in batch-Fahrweise als auch in einer quasikontinuierlichen Apparatur, wie sie in der US-PS 2985589 (1961) beschrieben ist, realisiert werden. Bei diesem, in dieser US-PS beschriebenen Chromatographie-Verfahren, wird die Stelle, bei der der "Feed"-Strom in das Trennharz eintritt, und die Stelle, bei der der "Produkt"-Strom aus dem Trennharz austritt, ständig verändert. Durch diese Schaltvorgänge wird eine gegenläufige Bewegung des Harzes simuliert, weshalb dieses Verfahren in der Literatur als "simulated moving bed"-Prozeß bezeichnet wird. Eine mögliche Realisierung dieses Prozesses, unter Einbeziehung der für das erfindungsgemäße Verfahren erforderlichen "Vorsäulen" wird in Fig. 9 dargestellt, die eine schematische Darstellung einer halbkontinuierlichen Chromatigraphieanlage zur Gewinnung reiner Milchsäure, mit Vor- und Trennsäulen, nach dem Prinzip des "simulated moving bed"-Prozesses angeordnet und geschaltet, zeigt.

Im Prinzip läuft auf dieser Anlage der Trennungs- und Reinigungsprozeß genau so ab wie oben geschildert, nur mit dem Unterschied, daß hier halbkontinuierlich gefahren werden kann. Auch hier wird auf eine der "Vorsäulen" eine bestimmte Menge "Feed" aufgebracht und mit Wasser nach unten gespült. Nach einer bestimmten Zeit wird auf die nächste "Vorsäule" eine weiter "Feed"-Menge aufgebracht und so ein kontinuierlicher "Feed"-Strom erzeugt. Das von der "Vorsäule" kommende, entkationisierte "Feed" wird mit einer Pumpe (P) direkt auf eine bestimmte "Trennsäule" befördert, während das Harz in dieser "Vorsäule" mit Schwefelsäure regeneriert wird. Dieses zyklische Beladen und Regenerieren der "Vorsäulen" kann nun beliebig oft wiederholt werden.

Die "Trennsäulen" hingegen sind durch eine Rohrleitung zu einem Ring verbunden. Diese Ringleitung ist zwischen den Säulen über Ventile an vier Versorgerleitungen angeschlossen. Diese Versorgerleitungen ermöglichen vier Prozeß-Ströme:

*) den "Feed"-Einlaßstrom
*) den "Eluent"-Einlaßstrom
*) den "Produkt"-Auslaßstrom, und
*) den "Raffinat"-Auslaßstrom

Der Fluß in der Anlage wird durch eine eigene Pumpe konstant gehalten. In diesem Prozeß sind nun immer genau vier Ventile geöffnet und teilen den aus Säulen gebildeten Ring in vier Zonen:

Die erste Zone ist die sog. "Adsorptionszone" und liegt zwischen dem Punkt, an dem der "Feed"-Strom in die "Trennsäulen" eintritt und jenem, an dem die schneller laufenden Substanzen als "Raffinat"-Strom abgezogen werden.

Die zweite Zone ist die sog. "Reinigungszone" und liegt zwischen den Punkt, an dem der "Raffinat"-Strom die "Trennsäulen" verläßt und jenem, an dem reines Wasser als "Eluent"-Strom in die "Trennsäulen" eintritt.

Die dritte Zone ist die sog. "Desorptionszone" und liegt zwischen den Punkt, an dem der "Eluent"-Strom in die "Trennsäulen" eintritt und jenem, an dem die Milchsäure als "Produkt"-Strom abgezogen wird.

Die Essigsäure und andere langsam laufenden Komponenten (Raffinatfraktion II), werden ebenfalls mit dem "Produkt"-Strom abgezogen, aber gesondert aufgefangen.

Die vierte Zone ist die sog. "Puffer-Zone" und liegt zwischen dem Punkt, an dem der "Produkt"-Strom die "Trennsäule" verläßt und jenem, an dem der "Feed"-Strom wieder in das Trennsystem eintritt.

Diese Zonen werden nun mit Hilfe eines Prozeßrechners nach vorgegeben Zeiten um eine Säule in Flußrichtung weiter geschaltet. Diese Schaltvorgänge simulieren einen Harzfluß gegen die Flußrichtung.

Der "simulated moving bed"-Prozeß hat gegenüber der batch-Fahrweise den Vorteil, daß für die Produktion derselben Menge an Milchsäure um etwa 1/3 weniger Harz, und um etwa 2/3 weniger Eluent benötigt wird.

Die Erfindung wird im folgenden an Hand von Beispielen näher erläutert. Die dabei verwendeten Harze sind übliche Handelsprodukte. Ihre, vom Hersteller angegebenen allgemeinen Eigenschaften sind in Tabelle 1 zusammengefäßt:

Tabelle 1

| Merkmal | Harz der Vorsäulen | | Harz der Trennsäulen |
|---|---|---|---|
| | Dowex MWC-1 | Lewatit MDS 1368 | Dowex Mono C 356 CA |
| Hersteller | Dow Chemical | Bayer | Dow Chemical |
| Harz-Typ | schwach sauer | stark sauer | stark sauer |
| Polymer-Basis | Polyacrly makroporös | Styrol/DVB gelförmig | Styrol/DVB gelförmig |
| Funktionelle Gruppe | Carboxylgruppe | Sulfonsäure | Sulfonsäure |
| Korngröße | 0,4-1,2 mm | 0,35 mm ±0,05 | 0,35 mm ±0,05 |
| Feuchtegehalt | 44-50 Gew.% | ca. 49 Gew.% | 57-61 Gew.% |
| Schüttdichte | 720 g/l | 830 g/l | 833 g/l |
| Totalkapazität | 3,8 val/l | 1,8 val/l | 1,5 val/l |
| max.Betriebstemp. | 100° C | 100° C | 90° C |
| pH-Arbeitsbereich | 5-14 | 1-14 | 1-14 |

Die bei allen IAC-Versuchen verwendete "Feed"-Lösung war eine durch Fermentation mit Lactobacillus delbrückii hergestellte Milchsäuremaische. Als Neutralisationsmittel wurde $NH_4OH$ verwendet, sodaß in der Maische die Milchsäure als Ammonium-Lactat vorlag. Nach Abtrennen der Biomasse mit einer Kammerzentrifuge wurde die Maische durch Vakuumverdampfung auf ca. 30 Gew.-Vol.-% aufkonzentriert und anschließend durch Zugabe von Aktivkohle entfärbt. Bei einem Teil der so hergestellten Maische wurde noch der pH-Wert durch Zugabe von konz. Schwefelsäure auf 2,5 gesenkt, und damit die Milchsäure aus ihrem Salz gedrängt.

Die auf diese Weise erhaltenen hellgelben Lösungen werden in den folgenden Beispielen mit "Feed"-Lösung 1 (pH 5,8) und "Feed"-Lösung 2 (pH 2,5) bezeichnet.

Die mit der HPLC (Shimadzu Co.) ermittelte Zusammensetzung dieser Lösungen wird in Tabelle 2 dargestellt.

Tabelle 2

| Analytische Charakterisierung der Fermentationslösung mit der HPLC | | |
|---|---|---|
| Substanz | Feed-Lösung 1 Ferm. Lösung bei pH 5,8 | Feed-Lösung 2 Ferm. Lösung bei pH 2,5 |
| Sulfate | 5,12 g/l | 124,20 g/l |
| Oxalsäure | 1,07 g/l | 0,97 g/l |
| Maltose | 4,68 g/l | 4,25 g/l |
| Glucose | 1,99 g/l | 1,79 g/l |
| Mannit | 4,64 g/l | 4,22 g/l |
| unbe.Substanz A | 2,91 g/l | 2,62 g/l |
| unbe.Substanz B | 2,28 g/l | 2,05 g/l |
| Milchsäure | 303,93 g/l | 268,40 g/l |
| Essigsäure | 2,80 g/l | 2,52 g/l |

Beispiel 1:

Eine Chromatographie-Versuchsanlage, bestehend aus zwei hintereinander geschalteten Doppelmantelsäulen aus Acrylglas, wurden mit dem stark sauren Kationenaustauscher DOWEX Mono C 356 CA gefüllt. Der Durchmesser der Innensäule betrug 2,0 cm und die Länge jeder Säule 200 cm. Bei einer Harzbetthöhe von 365 cm ergab sich daraus ein Harz-Bettvolumen von 1150 cm$^3$.

Nachdem die Säulen mit dem Harz gefüllt waren, wurden sie zuerst mit einer 2 n HCl in die H$^+$-Form gebracht und anschließend mit einem ca. 5 % Ammoniumwasser in die NH$_4^+$-Form umgeladen. Sämtliche bei dem Versuch auf die Säule aufgegebenen Flüssigkeitsvolumina wurden vorher entgast und auf die Säulentemperatur vorgewärmt. Diese betrug 55° C und wurde mit Umwälzthermostaten konstant gehalten.

Nach Aufgabe von 100 ml "Feed"-Lösung 1 auf die erste Säule wurde mit entgastem, entionisiertem Wasser, welches auf ca. 60° C vorgewärmt war, eluiert. Die Durchflußrate von 6 ml/min (1,91 ml/min*cm$^2$) wurde mit einer Schlauchquetschpumpe eingestellt und konstant gehalten. Das aus der ersten Säule unten austretende Eluat wurde unmittelbar auf die zweite Säule weitergepumpt. Das Eluat der zweiten Säule wurde durch eine Leitfähigkeitsmeßzelle geleitet und anschließend in einem Fraktionskollektor gesammelt. Das von der Leitfähigkeitsmeßzelle kommende Signal wurde von einem Schreiber in Form eines Leitfähigkeits-Chromatogamm aufgezeichnet. Es diente nur zur groben Detektion, da mit der Leitfähigkeit nur die Lage der Salze und Säuren erkannt werden kann.

Die genauere Untersuchung der gesammelten Fraktionen wurde mit der HPLC vorgenommen. Damit konnten nicht nur die Säuren und Salze, sondern auch die Kohlenhydrate quantitativ erfaßt werden.

Die mit der HPLC ermittelten Konzentrationen der einzelnen Substanzen wurde gegen das Eluatvolumen aufgetragen und ergab das in Fig. 1 dargestellte Chromatogramm.

Beispiel 2:

Beispiel 1 wurde wiederholt mit dem Unterschied, daß der stark saure Kationenaustauscher mit einer 2 n Schwefelsäure in die H$^+$-Form übergeführt und auf das Harz 100 ml der "Feed"-Lösung 2 aufgebracht wurde. Das Chromatogramm dieser Trennung wird in Fig. 2 dargestellt.

Beispiel 3:

Beispiel 2 wurde wiederholt mit dem Unterschied, daß die Chromatographieanlage um zwei weitere Säulen vergrößert wurde, wodurch sich bei eine Harzbetthöhe von 730 cm ein Harzbettvolumen von ca. 2300 cm$^3$ ergab. Auf das Harz wurden bei diesem Versuch nur 60 ml der "Feed"-Lösung 2 aufgegeben. Die Elution erfolgte nun so, daß nachdem die "Feed"-Lösung mit 40 ml Wasser in das Harzbett gespült war, mit 180 ml einer 2 n H$_2$SO$_4$ nachgefahren wurde. Anschließend wurde wieder auf Wasser umgeschaltet und wie in Beispiel 1 beschrieben, die Elution zu Ende geführt. Das Chromatogramm dieser Trennung wird in Fig. 3 dargestellt.

Beispiel 4:

Beispiel 3 wurde wiederholt mit dem Unterschied, daß auf das Harz 60 ml der "Feed-Lösung 1 aufgetragen wurden. Das Chromatogramm dieser Trennung wird in Fig. 4 dargestellt.

Beispiel 5:

Beispiel 4 wurde wiederholt mit dem Unterschied, daß die Chromatographie-Anlage um eine "Vorsäule" erweitert wurde. Diese "Vorsäule" hatte, bei einen Durchmesser von 2,5 cm und einer Harzbetthöhe von 80 cm, ein Harzbettvolumen von ca. 400 cm$^3$. Die "Vorsäule" wurde mit dem schwach sauren Kationenaustauscher Dowex MWC-1 gefüllt und mit einer 1 n H$_2$SO$_4$ in die H$^+$-Form gebracht. In der "Vorsäule" wurde mit einem Umwälzthermostaten eine Temperatur von 75° C aufrecht erhalten. Auf die "Vorsäule" wurden dann 60 ml "Feed"-Lösung 1 aufgetragen und mit vorgewärmten (70° C), entionisiertem Wasser mit einer Durchflußrate von 9 ml/min (2,8 ml/min*cm$^2$) eluiert. Das Chromatogramm dieser Trennung wird in Fig. 5 dargestellt.

Die Milchsäurefraktion wurde dem sog. "Hitze"-Test unterworfen. Dabei wurde eine Probe dieser Fraktion in einem druckfesten verschließbaren Röhrchen, im Glycerinbad einer 1-stündigen Erhitzung von 200° C ausgesetzt. Die Extinktion vor und nach der Erhitzung wurde bei 420 nm (Schichtdicke 1 cm) bestimmt.

Tabelle 3

| Merkmal | Extinktion |
| --- | --- |
| "Feed"-Lösung 1 | 0,412 |
| Probe von der Erhitzung | 0,006 |
| Probe nach der Erhitzung | 0,014 |

EP 0 684 941 B1

Beispiel 6:

Beispiel 5 wurde wiederholt mit dem Unterschied, daß die "Vorsäule" mit dem stark sauren Kationenaustauscher Lewatit MDS 1368 gefüllt und mit einer 2 n $H_2SO_4$ in die $H^+$-Form gebracht wurde. Das Chromatogramm dieser Trennung wird in Fig. 6 dargestellt.

Beispiel 7:

Der in Beispiel 5 mit $NH_4^+$-Ionen beladene, schwach saure Kationenaustauscher Dowex MWC-1 in der "Vorsäule" wurde mit einer 1 n $H_2SO_4$ regeneriert. Die aus der "Vorsäule" austretende Lösung lief durch eine Leitfähigkeitsmeßzell und wurde anschließend in einem Fraktionskollektor aufgefangen. Die Konzentration des Ammoniumsulfats in den einzelnen Fraktionen wurde durch Zugabe von konz. NaOH, und anschließender Wasserdampfdestillation mit der Büchi "Destillier-Einheit", bestimmt. Das Ergebnis dieses Versuches ist in Fig. 7 dargestellt.

Beispiel 8:

Beispiel 7 wurde wiederholt mit dem Unterschied, daß der in Beispiel 6 mit $NH_4^+$-Ionen beladene stark saure Kationenaustauscher Lewatit MDS 1368 in der "Vorsäule" mit einer 1 n $H_2SO_4$ regeneriert wurde. Das Ergebnis dieses Versuches ist in Fig. 8 dargestellt.

**Patentansprüche**

1. Verfahren zur Abtrennung und Reinigung von Milchsäure aus salz- und kohlenhydrathaltigen, von grobdispersen und lipophilen Verunreinigungen befreiten Substraten (Fermentationslösung) wobei der Abtrennungs- und Reinigungsprozeß die Kombination der folgenden zwei Schritten umfaßt:

   a) im ersten Schritt wird, in einer bder mehreren "Vorsäulen", das in der Fermentationslösung gegebenenfalls vorhandene Salz der Milchsäure durch einen echten Ionenaustausch in die freie Säure umgewandelt und
   b) im zweiten Schritt wird ebenfalls, in einer oder mehrerer "Trennsäulen", die freie Milchsäure durch Chromatographie an stark sauren Ionentauscherharzen von den übrigen in der Fermentationslösung vorhandenen Säuren, Kohlenhydraten und sonstigen Verunreinigungen getrennt.

2. Verfahren nach Anspruch 1, dadurch gekennzeichnet, daß die Fermentationslösung, aus welcher die Milchsäure abgetrennt wird, einen pH-Wert über 5,0 aufweist.

3. Verfahren nach Anspruch 1, dadurch gekennzeichnet, daß die Temperatur der "Vorsäule" mindestens 50° C, vorzugsweise jedoch eine Temperatur von 70° - 80° C beträgt.

4. Verfahren nach Anspruch 1, dadurch gekennzeichnet, daß der in einer oder in mehreren "Trennsäulen" befindliche stark saure Ionentauscherharz für die Chromatographie in der $H^+$-Form vorliegt.

5. Verfahren nach Anspruch 1, dadurch gekennzeichnet, daß die entkationisierte Fermentationslösung durch den Kontakt am stark sauren Ionentauscherharz und nachfolgende Eluierung in eine, die nichtmilchsäurehältigen Bestandteile enthaltene Raffinatfraktion I (Vorlauf), eine milchsäurehältige Produktfraktion und eine vornehmlich essigsäurehältige Raffinatfraktion II (Nachlauf) aufgetrennt wird.

6. Verfahren nach einen der Ansprüche 1 bis 4, dadurch gekennzeichnet, daß als Elutionsmittel zum Herauswaschen der einzelnen Fraktionen aus den Trennsäulen reines, vorzugsweise entionisiertes Wasser verwendet wird.

7. Verfahren nach Anspruch 5, dadurch gekennzeichnet, daß die Elutionstemperatur zwischen der Raumtemperatur und der Stabilitätsgrenztemperatur der verwendeten Harze, vorzugsweise bei 50°-65°C liegt.

8. Verfahren nach Anspruch 1, dadurch gekennzeichnet, daß nur der in den "Vorsäulen" befindliche schwach saure Kationenaustauscher nach seiner vollkommenen Beladung mit den Kationen der Fermentationslsöung mit einer verdünnten starken Mineralsäure, vorzugsweise mit einer 1 - 2 n Schwefelsäure regeneriert wird.

9. Verfahren nach Anspruch 7, dadurch gekennzeichnet, daß die bei der Regeneration der Harze in den Vorsäulen anfallende verdünnte Salzlösung durch salzspaltende Elektrodialyse in die korrespondierenden Säuren und Basen gespalten und wieder in den Prozeß zurückgeführt werden.

## Claims

1. A method of isolating and purifying lactic acid from substrates containing salt and carbohydrates and cleansed of coarsely disperse and lipophilic impurities (fermentation solution), the isolating and purification process comprising the combination of the following two steps:

   a) in the first step, in one or more "preliminary columns", the salt of lactic acid possibly present in the fermentation solution is converted by a genuine ion exchange into the free acid and
   b) in the second step, likewise in one or more "separating columns", the free lactic acid is isolated from the other acids, carbohydrates and other impurities present in the fermentation solution by chromatography on strongly acid ion exchange resins.

2. A method according to claim 1, characterised in that the fermentation solution from which the lactic acid is isolated has a pH value of more than 5.0.

3. A method according to claim 1, characterised in that the temperature of the "preliminary column" is at least 50°C but is preferably a temperature of 70° to 80°C.

4. A method according to claim 1, characterised in that the strongly acid ion exchange resin for the chromatography and present in one or a plurality of "separating columns" is in the H+ form.

5. A method according to claim 1, characterised in that the decationised fermentation solution is separated by contact with the strongly acid ion exchange resin and subsequent elution into a refinate fraction I containing the constituents which do not comprise lactic acid (first runnings) a product fraction containing lactic acid and a refinate fraction II containing mainly acetic acid (tailings).

6. A method according to one of claims 1 to 4, characterised in that pure preferably deionised water is used as an eluting agent for washing the individual fractions out of the separating columns.

7. A method according to claim 5, characterised in that the elution temperature is between room temperature and the stability limit temperature of the resins used, being preferably around 50° to 65°C.

8. A method according to claim 1, characterised in that only the weakly acid cation exchanger present in the "preliminary columns" is, after it is completely charged with the cations from the fermentation solution, regenerated with a dilute strong mineral acid, preferably a 1 to 2n sulphuric acid.

9. A method according to claim 7, characterised in that the dilute salt solution arising during regeneration of the resins in the preliminary columns is broken down into the corresponding acids and bases by salt-splitting electrodialysis and fed back into the process.

## Revendications

1. Procédé de séparation et de purification d'acide lactique dans des substrats (solution de fermentation) contenant du sel et des hydrates de carbone, débarrassés d'impuretés en dispersion grossière et lipophiles, le processus de séparation et de purification comprenant la combinaison des deux étapes suivantes :

   a) dans la première étape, dans une ou plusieurs "pré-colonnes", le sel de l'acide lactique éventuellement présent dans la solution de fermentation est transformé en l'acide libre par un échange réel d'ions, et
   b) dans la deuxième étape, également dans une ou plusieurs "colonnes de séparation", l'acide lactique libre est séparé du restant des acides, des hydrates de carbone et des autres impuretés, présents dans la solution de fermentation, par chromatographie sur des résines échangeuses d'ions fortement acides.

2. Procédé selon la revendication 1, caractérisé en ce que la solution de fermentation de laquelle l'acide lactique est séparé présente une valeur de pH de plus de 5,0.

3. Procédé selon la revendication 1, caractérisé en ce que la température de la "pré-colonne" est d'au moins 50°C, s'élevant toutefois, de préférence, à une température de 70°C à 80°C.

4. Procédé selon la revendication 1, caractérisé en ce que la résine échangeuse d'ions fortement acide se trouvant dans une ou dans plusieurs "colonnes de séparation" pour la chromatographie se présente sous la forme H⁺.

5. Procédé selon la revendication 1, caractérisé en ce que la solution de fermentation décationisée, par le contact avec la résine échangeuse d'ions fortement acide suivi d'une élution, est séparée en une fraction de produit raffiné I (premier produit) contenant les composants ne contenant pas d'acide lactique, une fraction de produit contenant de l'acide lactique, et une fraction de produit raffiné II (fraction de queue) contenant principalement de l'acide acétique.

6. Procédé selon l'une des revendications 1 à 4, caractérisé en ce que l'on utilise comme agent éluant pour l'extraction des différentes fractions hors des colonnes de séparation, de l'eau pure, de préférence désionisée.

7. Procédé selon la revendication 5, caractérisé en ce que la température d'élution se situe entre la température ambiante et la température limite de stabilité des résines utilisées, de préférence entre 50°C et 65°C.

8. Procédé selon la revendication 1, caractérisé en ce que seul l'échangeur de cations faiblement acide se trouvant dans les "pré-colonnes" est régénéré, après son alimentation complète avec les cations de la solution de fermentation, à l'aide d'un acide minéral fort dilué, de préférence à l'aide d'un acide sulfurique de 1 à 2 n.

9. Procédé selon la revendication 7, caractérisé en ce que la solution saline diluée qui se retrouve dans les pré-colonnes lors de la régénération des résines, est décomposée par électrodialyse décomposant le sel, en les acides et les bases correspondants, puis à nouveau réintroduite dans le processus.

IA-Chromatographie der "Feed"-Lösung 1
mit Hilfe von Dowex Mono C 356 CA
stark saurer Kationenaustauscher in der NH4-Form

Legend:
- ⧫ - Salze (SO4)    - + - Oxalsäure    - × - Maltose    - ▽ - Glucose
- ◇ - Mannit    - △ - unbe.Subs.(B)    - ▣ - Milchsäure    - ◆ - Essigsäure

Konz. (Milchsäure), g/l

Konz. (SO4;Oxa;Mal;Glu;Man;ubS(B);Essigs;) g/l

Eluatvolumen in ml

Fig. 1

EP 0 684 941 B1

IA-Chromatographie der "Feed"-Lösung 2
mit Hilfe von Dowex Mono C 356 CA
stark saurer Kationenaustauscher in der H–Form

Fig. 2

Fig. 3

IA-Chromatographie der "Feed"-Lösung 1
mit Hilfe von Dowex Mono C 356 CA
stark saurer Kationenaustauscher in der H—Form

Fig. 4

EP 0 684 941 B1

IA-Chromatographie der "Feed"-Lösung 1 mit Hilfe von
DOWEX MWC-1 (Vorsäule) und DOWEX Mono C 356 CA (Trennsäule)
schwach und stark saure Kationenaustauscher in der H–Form

Fig. 5

IA-Chromatographie der "Feed"-Lösung 1 mit Hilfe von
LEWATIT MDS 1368 (Vorsäule) und DOWEX Mono C 356 (Trennsäule)
beide sind stark saure Kationenaustauscher in der H−Form

Fig. 6

Regeneration des schwach sauren
Kationenaustauschers Dowex MWC-1 in der "Vorsäule"
mit 1 n H2SO4 bei 55 grd C

—◇— Leitfähigkeit    —□— (NH4)2SO4

Leitfähigkeit in mS/cm

Konzentration (NH4)2SO4 in g/l

Eluatvolumen (1 n HCl) in ml

Fig. 7

EP 0 684 941 B1

Regeneration des stark sauren
Kationenaustauschers Lewatit MDS-1368 in der "Vorsäule"
mit 1 n H2SO4 bei 55 grd C

Konzentration (NH4)2SO4 in g/l

Leitfähigkeit in mS/cm

Eluatvolumen (1 n HCl) in ml

Leitfähigkeit    (NH4)2SO4

Fig. 8

22

Fig. 9